# EUROPEAN PATENT APPLICATION

(11) **EP 2 191 835 A1**
(43) Date of publication of application: **02.06.2010**
(21) Application number: 08829164.6
(22) Date of filing: 04.09.2008
(51) Int. Cl.: A61K 35/28, A61P 19/02, A61P 29/00

(54) **THERAPEUTIC AND PROPHYLACTIC AGENTS FOR ARTHRITIS**

(30) Priority: 07.09.2007 JP 2007233094
(71) Applicant: JCR Pharmaceuticals CO., LTD., Ashiya-shi, Hyogo 659-0021 (JP); Miracure Inc., Tokyo 191-0002 (JP)
(72) Inventor: KURODA, Masahiko, Tokyo 167-0052 (JP); TAKANASHI, Masakatsu, Isehara-shi Kanagawa 259-1141 (JP); SUDO, Katsuko, Asaka-shi Saitama 351-0012 (JP); YAMAUCHI, Shigeki, Kawasaki-shi Kanagawa 211-0065 (JP); SHIRONO, Hiroyuki, Kobe-shi Hyogo 651-2243 (JP); HIRADO, Toru, Sanda-shi Hyogo 669-1321 (JP); MAEDA, Kenichi, Kyoto-shi Kyoto 606-8304 (JP)
(74) Representative: Behnisch, Werner
(86) International application number: PCT/JP2008/065943
(87) International publication number: WO 2009/031606

(57) **Abstract**

Provided is a pharmaceutical composition and a method for the treatment and/or prophylaxis of arthritis, inter alia, rheumatoid arthritis. The pharmaceutical composition comprises human mesenchymal stem cells, and the method comprises administering an effective amount human mesenchymal stem cells to a patient.

## Description

### TECHNICAL FIELD

The present invention relates to a pharmaceutical composition for the treatment and prophylaxis of arthritis and, in particular, a pharmaceutical composition for the treatment and prophylaxis of arthritis containing as an active ingredient human mesenchymal stem cells, and among others, to a pharmaceutical composition for the treatment and prophylaxis of rheumatoid arthritis.

### BACKGROUND ART

Rheumatoid arthritis is a non-purulent, multiple chronic arthritis. This is a disease with a high incidence, number of the patients with this disease being about 5 million in the world, and there are as many as about 700 thousand patients in Japan only. Usually, rheumatoid arthritis first takes place at such joints that are located at distal part of the body, such as those of fingers and toes, and then the disease gradually affects bigger joints located at more central part of the body, like knee joints, and even the cervical vertebra. A joint affected by rheumatoid arthritis is accompanied, according to the degree of progression of the disease, by swelling, stiffening, and pains, and they bring about difficulties to the patient's daily life. As arthritis progresses, such events follow as destruction and loss of the cartilage, erosion of the bones, loss of the articular cavity, and deformation and dislocation of the joint, and, further, even the direct contact of the bones with each other (anchylosis) may be brought about as a result of loss of the joint structure itself.

In many cases, autoantibodies directed to the Fc region of IgG (rheumatoid factors: IgM, IgG, IgA, and IgE) are found positive in the blood of rheumatoid arthritis patients, which generally are particularly noticed in patients with severe disease. However, not a few cases are known in which the rheumatoid factors are negative in even though the patient does suffer rheumatoid arthritis. On the other hand, there are some cases in which the rheumatoid factors are found positive in patients suffering other diseases than rheumatoid arthritis, such as collagenosis, infectious diseases, chronic liver diseases or the like, and in aged people as well. Thus, the cause of rheumatoid arthritis, including how the rheumatoid factors are involved in the onset of the disease, is not known.

Histopathologically, rheumatoid arthritis begins with infiltration of inflammatory cells, such as neutrophils and lymphocytes in the tissues, into the articular cavity. Under excessive production of soluble factors such as inflammatory cytokines, hyperplasia of the synovial membrane occurs on the cartilage, which then, destroying the cartilage and infiltrating into the bones and destroying them, forms a villiform tissue (pannus) consisting of synovial cells, fibroblasts and the like. Along with the progression of the disease, the articular cavity becomes completely filled with hyperplastic synovial membranes and inflammatory cells (neutrophils, inflammatory lymphocytes, etc.). In this condition, loss of the cartilage and destruction of the bones have progressed, and the joint has already lost its movability.

In the current guiding principle for the treatment of rheumatoid arthritis, it is recommended that when a patient is diagnosed with rheumatoid arthritis, an antirheumatic drug, such as methotrexate, be administered without delay. Methotrexate, which is an inhibitor of folic acid synthesis and is recognized as a first-choice medicine as one of the standard drugs, is thought to exhibit an immunosuppressive effect through inhibition of nucleic acid synthesis, and thus such severe side effects including death have been reported as bone marrow suppression causing leukopenia. Further, severe cases of other side effects including death has also been reported with methotrexate, such as development of interstitial pneumonia. Due to the risk of these side effects, this drug has a drawback that it must be administered cautiously. Furthermore, it has other drawbacks: that the rate of the rheumatoid arthritis patients in whom the drug does not work reaches as much as 40 %, and that it takes no less than 4 weeks, even in the cases the drug proves effective, for the drug to exhibit its effect after administration.

On the other hand, while adrenocortical steroid preparations have immunosuppressive and antiinflammatory activities, and are effective in treating rheumatoid arthritis, they have disadvantages that their prolonged use leads to the development of gastrointestinal disorders, osteoporosis, moon face and the like as side effects, and also to decrease in the effect. Moreover, they have another disadvantage that due to the risk of rebound which could occur when their administration is discontinued, they makes it hard for the patients to withdraw from the drug.

Non-steroidal antiinflammatory drugs (NSAIDs) also are used to inflammation and pains in rheumatoid arthritis. However, this is nothing more than a symptomatic treatment, and it cannot suppress the progression of destruction of the bones in rheumatoid arthritis.

In such a situation, what has been drawing attention recently is biological preparations which work through inflammatory cytokines occurring in the regions affected by inflammation in rheumatoid arthritis. The most typical ones are TNF-α inhibiters, which are infliximab, adalimumab and etanercept. However, as it is a chimeric antibody, side effect problems have been pointed with infliximab, such as the emergence of a patient's antibody against it, which then causes reduction of the effect of the drug. In the case of adalimumab and etanercept, which are entirely human-type antibodies, though emergence of patient's antibodies against them is suppressed, their effect is insufficient when administered alone, and thus it is still recommended that those drugs are to be given concurrently with methotrexate. And any of these TNF-α inhibiters might lead to severe infectious diseases as side effects, such as tuberculosis including miliary tuberculosis and extrapulmonary tuberculosis and sepsis and the like, or further, lethal respiratory tract infections due to opportunistic infection.

Currently, as mentioned above, there is no drug which is both sufficiently effective and safe to be used for rheumatoid arthritis. On the other hand, the number of the rheumatoid arthritis patients is already huge, and continues to rapidly increase along with the aging in the population distribution in developed countries.

Besides rheumatoid arthritis, there is another arthritis, a chronic or refractory arthritis accompanying Still's disease. As for this arthritis accompanying Still's disease, like rheumatoid arthritis, the cause of the disease is unknown, though there are speculations that viral infection or immune anomaly is involved in some manners. Both non-steroidal antiinflammatory drugs and adrenocortical steroids which are used for rheumatoid arthritis are also used as therapeutics for treating arthritis accompanying Still's disease. However, they have drawbacks, e.g., that their therapeutic effect is not constant among patients. Thus, development of effective therapeutic drugs has been also hoped for which are effective for such chronic or refractory arthritis.

In regions affected by inflammation, such events are generally observed as destruction of the cartilage and bones of the joint, infiltration of inflammatory cells such as neutrophils and lymphocytes into the articular cavity, hyperplasia of the synovial membrane. These, however, are widely observed events not only in rheumatoid arthritis but also in arthritis in general, and are the common basic physiological features of arthritis. Drugs which suppress or block them would be useful in treating not only rheumatoid arthritis but various arthritis in general,

Mesenchymal stem cells (MSC) are undifferentiated cells very rarely found in mesenchymal tissues such as bone marrow. And they are multipotent and have, along with high proliferation potential, an ability to differentiate into bone cells, chondrocytes, muscle cells, tendon cells, stromal cells, adipose cells, and the like. Human mesenchymal stem cells can be grown in culture using an artificial culture medium (Patent Document 1), and it is known that they, like other cultured cells, can be stored and supplied in frozen state. It is also known that mesenchymal stem cells can be obtained not only from bone marrow but also from various other tissues such as adipose tissue (Patent Document 2), dental pulp cells (Patent Document 3), placenta tissue or umbilical cord tissue (Patent Document 4).

Disclosures have been made: that rejection of a skin graft between allogeneic baboons was suppressed by administering baboon mesenchymal stem cells to the recipient animals; that development of graft-versus-host disease (GvHD) after bone marrow transplant between allogeneic dogs was suppressed by administering canine mesenchymal stem cells; further that human T lymphocytes did not show proliferation response to human mesenchymal stem cells (allogeneic) in vitro (in mixed lymphocyte reaction); and that human mixed lymphocyte reaction was suppressed in MHC type non-specific manner by human mesenchymal stem cells; and that this was also the case in dogs. And, based on these results, a possibility is disclosed about the use of human mesenchymal stem cells to suppress immune response after transplantation between humans (see Patent Documents 5 and 6, both based on the same priority application, and Patent Document 7, which is a translation of the latter).

Expecting such effect of mesenchymal stem cells, a clinical trial was conducted to evaluate the suppressive effect of human mesenchymal stem cells on acute graft-versus-host disease (GvHD) after human bone marrow transplantation in steroid-resistant severe GvHD patients, and the results was reported (see Non-patent Document 1). According to this report, the patients were administered with anti-cancer drugs (cyclophosphamide, busulphan, fludarabin) and/or total body irradiation (TBI) against malignant tumors, and with methotrexate and cyclophosphamide or prednisolone for prevention and treatment of GvHD. And human mesenchymal stem cells (0.7 - 9 × 10⁶ cells/kg body weight) were administered thereafter. It is reported that in these patients with their immune system suppressed, 6 out of 8 patients responded to the administration of mesenchymal stem cells in total.

Though in connection with GvHD, mesenchymal stem cells is thought to bring about suppression of the host's immune system (see Non-patent Document 2), the mechanism of it is not fully understood. It has also been proposed to use mesenchymal stem cells to treat neovascularization, autoimmune diseases, inflammatory responses (in Alzheimer's disease, Parkinson's disease, stroke, brain cell injuries, psoriasis, chronic dermatitis, contact dermatitis, arthrosteitis, arthritis including rheumatoid arthritis, inflammatory bowel disease, chronic hepatitis), cancer, allergic diseases, sepsis, trauma (burn injury, surgery, transplantation), inflammation in various tissues or organs (cornea, lens, pigment epithelium, retina, brain, spinal cord, uterus during pregnancy, ovary, testis, adrenal gland) (see Patent Document 8). However, in this document, the function of mesenchymal stem cells was examined in vitro only, and no examination was made in vivo or using some particluar disease model. Therefore, the document provides no clue to evaluating actual usefulness of mesenchymal stem cells for such diseases.

On the other hand, an animal experiment-based report has been made, focusing attention on suppression of immune system, as to whether mesenchymal stem cells have therapeutic value to successfully suppressing active T cells in arthritis such as rheumatoid arthritis (see Non-patent document 3). According to this document, mouse mesenchymal stem cells (allogeneic) were administered (injection into the tail vein, 10⁶ or 4 × 10⁶ cells) to the mice that had been made to develop collagen-induced arthritis, a model of rheumatoid arthritis. And as the mouse mesenchymal stem cells did not bring about any benefit, but the arthritis was aggravated although no homing of the cells to the joint affected with inflammation was observed, it has been concluded that mesenchymal stem cells are not appropriate for use to treat arthritis, at least as they were.

On the other hand, it has been recently reported that administration of mouse mesenchymal stem cells (allogeneic) to collagen-induced arthritis mice (intraperitoneal, 5 × 10⁶ cells) ameliorated the symptoms of arthritis (see Non-patent Document 4). In this document, a reason is also stated why these results obtained which was contradictory to the results demonstrated in a previous document (Patent Document 3). In that statement, it is pointed out that the experiment described in the previous document had used mesenchymal cells which had been immortalized and made into a cell line (in contrast, in the latter report, primary culture cells, which was subcultured only once in vitro were used), and that the cell line mentioned in the previous document had not been effective in blocking antibody-specific immune response in vivo. Besides the differences mentioned above, however, there was another difference in the route of administration: the mesenchymal stem cells were injected into the tail vein in the former document, whereas in the latter, they were injected intraperitoneally. As mesenchymal stem cells are relatively large in size and somewhat adhesive, many of them are thought to be trapped by the peritoneum after intraperitoneally administered, and therefore hardly go into the circulating blood. Therefore such differences in the route of administration between the both reports is critical. In fact, the latter document reports that the administered mesenchymal stem cells formed colonies on the peritoneum over a week after the administration. Though it is also stated at the same time that those cells looked like beginning to move into the blood stream and that some reached the spleen 7 days after administration, leaving a trace there, the route of administration is in sharp contrast to intravenous injection, in which the mesenchymal stem cells can be directly introduced into the bloodstream without any concern about their being trapped by the peritoneum. Therefore, this difference in their distribution in the body after administration is considered to be one of the causes. In the previous document, it is reported that the homing of the intravenously administered mesenchymal stem cells to the joint affected with inflammation was not observed, and no mention is given to the homing to the joint in the latter document. A speculation is given to a mechanism in the latter document that high amounts of TNFα is produced by mesenchymal stem cells and this results in some "paradoxical" antiinflammatory effect. However, the reason is very unclear as to why intravenous injection produced no effect, but intraperitoneal injection did.

Thus, there are many factors whose influences are unknown, such as the route of administration or whether or not the cells employed are primary cultured or subcultured cells, and so on, as to whether or not administration of allogeneic mesenchymal stem cells could suppress arthritis in animals. In particular, there has been negative prospect as to whether or not arthritis could be suppressed if mesenchymal stem cells are intravenously administered, and among others, if subcultured mesenchymal stem cells, which are available with less difficulty, (as opposed to primary cultured cells) are employed and administered intravenously.

[Patent Document 1] United States Patent No. 5486359
[Patent Document 2] Japanese Patent Application Publication No. 2004-129549
[Patent Document 3] Japanese Patent Application Publication No. 2004-201612
[Patent Document 4] Japanese Patent Application Publication No. 2004-210713
[Patent Document 5] United States Patent No. 6328960
[Patent Document 6] WO 99/47163
[Patent Document 7] Japanese Patent Application Publication No. 2002-506831
[Patent Document 8] WO 2005/093044
[Non-patent Document 1] Transplantation, 81(10):1390-1397(2006)
[Non-patent Document 2] Blood, 105(4):1815-22(2005)
[Non-patent Document 3] Arthritis & Rheumatism, 52(5),1595-1603(2005)
[Non-patent Document 4] Arthritis & Rheumatism, 56(4):1175-1186(2007)

### DISCLOSURE OF THE INVENTION

### [Problem to be solved by the invention]

Against the above-mentioned background, the objective of the present invention is to provide a means to treat and/or prevent arthritis, inter alia rheumatoid arthritis.

### [Means to solve the problem]

The present inventors attempted to intravenously inject collagen-induced arthritis mice with rat mesenchymal stem cells, and found that no transplant rejection took place, and, even though the cells administered were allogeneic for the mice, the arthritis in the mice was suppressed (Examples, Experiment 3). The present inventors then intravenously injected collagen-induced arthritis mice with human mesenchymal stem cells (subcultured cells), and again found that the arthritis was notably suppressed even though the cells were allogeneic (Examples, Experiments 1 and 2). The present invention was completed on the basis of these discoveries.

Thus, the present invention provides what follows:
1. A pharmaceutical composition for the treatment and prophylaxis of arthritis comprising human mesenchymal stem cells.
2. The pharmaceutical composition for the treatment and prophylaxis of arthritis according to 1 above, wherein the arthritis is arthritis in a mammal.
3. The pharmaceutical composition for the treatment and prophylaxis of arthritis according to 2 above, wherein the mammal is a human or a rodent.
4. The pharmaceutical composition for the treatment and prophylaxis of arthritis according to 2 above, wherein the mammal is a human.
5. The pharmaceutical composition for the treatment and prophylaxis of arthritis according to 3 or 4 above, wherein the human mesenchymal stem cells are human bone marrow-derived cells.
6. The pharmaceutical composition for the treatment and prophylaxis of arthritis according to 5 above, wherein the mammal is a human, and wherein the human is not the same as the human from whom the human mesenchymal stem cells were derived.
7. The pharmaceutical composition for the treatment and prophylaxis of arthritis according to 5 or 6 above, wherein the mammal is a human, and wherein the arthritis is rheumatoid arthritis.
8. The pharmaceutical composition for the treatment and prophylaxis of arthritis according to one of 1 to 7 above, wherein the composition is an injection.
9. The pharmaceutical composition for the treatment and prophylaxis of arthritis according to 8 above, wherein the composition is an injection for intravenous administration.
10. A method for the treatment of arthritis in a mammalian patient, comprising administering to the patient a therapeutically effective amount of human mesenchymal stem cells.
11. The method for the treatment according to 10 above, wherein the mammal is a human or a rodent.
12. The method for the treatment according to 10 above, wherein the mammal is a human.
13. The method for the treatment according to one of 10 to 12 above, wherein the human mesenchymal stem cells are human bone marrow-derived cells.
14. The method for the treatment according to 13 above, wherein the mammal is a human, and wherein the arthritis is rheumatoid arthritis.
15. The method for the treatment according to one of 10 to 14 above, wherein the human mesenchymal stem cells are administered to the patient by injection.
16. The method for the treatment according to 15 above, wherein the human mesenchymal stem cells are intravenously administered.
17. Use of human mesenchymal stem cells for the production of a pharmaceutical composition for the treatment and prophylaxis of arthritis in a mammal.
18. The use according to 17 above, wherein the mammal is a human or a rodent.
19. The use according to 17 above, wherein the mammal is a human.
20. The use according to one of 17 to 19 above, wherein the human mesenchymal stem cells are human bone marrow-derived cells.
21. The use according to 20 above, wherein the mammal is a human, and wherein the arthritis is rheumatoid arthritis.

Human mesenchymal stem cells can be used for the production of a pharmaceutical composition for the treatment and/or prophylaxis of arthritis in a mammal, inter alia a human and rodent. The pharmaceutical composition for the treatment and prophylaxis of arthritis according to the present invention thus obtained can very strongly suppress the inflammation itself which consists of infiltration of inflammatory cells into the articular cavity and hyperplasia of the synovial membrane, and concurrent destruction and loss of the cartilage, erosion and destruction of the bones in arthritis in mammals, thereby ameliorating arthritis and preventing or delaying its progression. Therefore, the pharmaceutical composition of the present invention can be used as a medicament to treat inflammation itself, and thereby to prevent destruction of the joint in arthritis in a mammal, inter alia a human, and among others rheumatoid arthritis, which affects a number of patients, greatly damages their quality of life as it progresses to a severe stage, and even may turn out to be fatal.

The pharmaceutical composition for the treatment and prophylaxis of arthritis according to the present invention exhibits a remarkable effect when administered only a very limited number of times, such as a single administration, and has also an advantage that the effect of it is quickly obtained. Furthermore, in spite that the pharmaceutical composition according to the present invention is a preparation containing cells, human mesenchymal stem cells, the active ingredient, do not invoke immune responses in an allogeneic host. Thus, it can be administered beyond the borders between species, which is impossible with general cells without concurrent application of immunosuppressive means. And in human, too, it can be administered without paying attention to the difference in MHC (major histocompatibility complex) between the patient and the mesenchymal stem cells contained in the pharmaceutical composition according to the present invention for the prevention and prophylaxis of arthritis. Therefore, the pharmaceutical composition containing human mesenchymal stem cells according to the present invention may be stocked as a composition produced and provided in advance, and the same composition may be applied to any patient in common. Thus, although the pharmaceutical composition for the treatment and prophylaxis of arthritis according to the present invention is a cell-based pharmaceutical composition, it will not impose a physical burden and risk on the patient of collecting cells from his or her own bone marrow because there is no need to take the cells from the very patient to be treated. Therefore, the method for the treatment of arthritis by administering human mesenchymal stem cells to a patient with arthritis is highly useful as a method for the treatment of arthritis, inter alia human arthritis, and among others, rheumatoid arthritis.

### BRIEF DESCRIPTION OF DRAWINGS

[FIG. 1] A graph showing the incidence of arthritis in Experiment 1.
[FIG. 2] A photograph showing the external appearance of a hindlimb of (a) a mouse of the control group which has developed arthritis and (b) a mouse of the MSC-administration group which has not developed arthritis in Experiment 1.
[FIG. 3] A graph showing the change in the mean RA scores obtained for the MSC-administration group and the control group in Experiment 1.
[FIG. 4] A graph showing the change in the mean RA scores obtained for the MSC-administration-before-antibody group, the MSC-administration-before-LPS group, the MSC-administration-after-LPS group, and the control group in Experiment 2.
[FIG. 5] A graph showing the incidence of arthritis in Experiment 3.
[FIG. 6] A graph showing the change in the mean RA scores obtained for the MSC-administration group and the control group in Experiment 3.
[FIG. 7a] A photograph (magnification: × 20) showing a tissue section taken from a joint of a normal mouse in Experiment 1. The upper is the original picture and the lower the same picture with comments on it. Broken lines show the borders of tissues or distinct regions (the same is applicable hereinafter).
[FIG. 7b] A photograph of a tissue section taken from a joint of a mouse of the control group in Experiment 1 (magnification: × 20).
[FIG. 7c] A photograph of a tissue section taken from a joint of a mouse of the MSC-administration group which had not developed arthritis in Experiment 1 (magnification: × 20).
[FIG. 7d] A photograph of a tissue section taken from a joint of a mouse of the MSC-administration group which had developed arthritis in Experiment 1 (magnification: × 20).
[FIG. 8a] A photograph of a tissue section taken from the site of inflammation in a joint of a mouse of the control group in Experiment 2 (magnification: × 10).
[FIG. 8b] A photograph of a tissue section taken from the site of inflammation in a joint of a mouse of the MSC-administration-before-antibody group in Experiment 2 (magnification: × 20).
[FIG. 8c] A photograph of a tissue section taken from the site of inflammation in a joint of a mouse of the MSC-administration-before-LPS group in Experiment 2 (magnification: × 20).
[FIG. 8d] A photograph of a tissue section taken from the site of inflammation in a joint of a mouse of the MSC-administration-after-LPS group in Experiment 2 (magnification: × 20).
[FIG. 9a] A photograph of a tissue section taken from the site of inflammation in a joint of a mouse of the control group in Experiment 3 (magnification: × 20).
[FIG. 9b] A photograph of a tissue section taken from the site of inflammation in a joint of a mouse of the MSC-administration group in Experiment 3 (magnification: × 20).

### [Explanation of signs]

- A: Articular cavity
- B: Articular cartilage (hyaline cartilage)
- C: Chondrocyte
- D: Bone matrix (cancellous bone)
- E: Bone marrow
- F: Sinusoid
- G: Supporting tissue
- H: Connective tissue (muscle, etc.)
- I: Site of hyperplasia of granulation tissue (including synovial cells, fibroblasts)
- J: Site of infiltration of inflammatory cells (including polymorphonuclear granulocytes (neutrophils, acidophils), lymphocytes)
- K: Cutaneous tissue
- L: Adipose cells
- M: Hyperplasia of osteoclast
- N: Increased reactivity of osteoblast
- O: Site of hyperplasia of synovial membrane
- ★: Hematopoietic stem cells
- *: Blood cells

### BEST MODE FOR CARRYING OUT THE INVENTION

The pharmaceutical composition for the treatment and prophylaxis of arthritis according to the present invention contains as an active ingredient human mesenchymal stem cells. In the present invention, though human mesenchymal stem cells may be mesenchymal stem cells obtained from human tissues, such as bone marrow, adipose tissue, dental pulp cells, placenta tissue, and the like, bone marrow-derived mesenchymal stem cells are particularly preferred. A cell line of mesenchymal stem cells derived from human bone marrow (Poietics™, Cambrex Bio Science Walkersville, Inc., MD, USA) are commercially available already, which may be used, either directly or after subcultured.

In the present invention, human mesenchymal stem cells have not to be primary culture cells, but subcultured cells may be used. Though primary culture cells can be used because they are by no means inferior to subcultured cells, subcultured cells are generally used in order to meet the practical need that they should be produced, stored and used in substantial amounts as products. In the present invention, the active ingredient, the human mesenchymal stem cells, may, for example, be supplied in frozen state, and thawed just before its administration, then suspended in an aqueous medium and administered. Alternatively, however, it is also allowed that, for example, cultured human mesenchymal stem cells are collected, suspended without being subjected to freezing, and then administered. In these processes, an aqueous medium in which to suspend the cells may be, for example, an aqueous solution for injection, as desired, whose osmotic pressure and pH are adjusted to or near the values of the blood, and which is adjusted with regard to the content of salts. For example, but without limitation, intravenous fluids including Ringer's solutions such as acetated Ringer's solution, and, glucose acetated Ringer's solution, as well as physiological saline, or glucose solution may be used. When Ringer's solution for infusion is used, an acceptable amount of dimethylsulfoxide (DMSO) or human serum albumin (HSA) may be added to it.

In the present invention, human mesenchymal stem cells may be administered in the form of an injection intravenously, intramuscularly, or subcutaneously, among which intravenous administration is preferred. When conducting intravenous administration, the injection may either be administered directly to the patient from a syringe or, in the manner of intravenous drip, after once added to the intravenous fluid in a drip infusion bag, administered from this bag to the patient. Further, human mesenchymal stem cells may be administered directly to the affected site from a syringe. In the present invention, the term "patient" includes human and non-human mammals.

As to the manner of administration of the mesenchymal stem cells, they may be administered either once or two or more times. The frequency and timing of administration may be adjusted as desired according to the symptoms observed in the patient.

In the present invention, the pharmaceutical composition for the treatment and prophylaxis of arthritis may be administered not only to patients who have already developed arthritis but also to patients who have not yet developed arthritis but are at high risk of developing it. Patient who are at high risk of developing arthritis can be screened, for example, based on their history of disease or by measurement of the level of rheumatoid factors in the blood. Also as for intermediate cases falling between the above two, i.e., in such cases where an early stage of arthritis is suspected or arthritis is once diagnosed to have cured, it is beneficial to administer the pharmaceutical composition for the treatment and prophylaxis of arthritis, in order to prevent onset or recurrence of arthritis in patients. This is because arthritis often progresses with repeated amelioration and aggravation, and accurate and proper diagnosis of it is difficult when symptoms are very mild as is the case at its early stages, and thus there is a possibility that inflammation already exists even if it cannot be detected from outside of the body, and because it is of great therapeutic importance to treat arthritis at its early stages and thereby block its progress.

The pharmaceutical composition for the treatment and prophylaxis of arthritis according to the present invention is a preparation for use for any patient in common, it is generally administered to patients who are allogeneic to it. However, it is not prohibited to administer it to syngeneic patients (e.g., one of the twins from the other of whom the mesenchymal stem cells have been collected), nor is it prohibited that the pharmaceutical composition for the treatment and prophylaxis of arthritis according to the present invention, which is produced as a preparation for use for any patient in common, happens to be administered to the human who is the very source from which the mesenchymal stem cells were collected, as autologous cells to treat and prevent arthritis.

When administered, the density of the human mesenchymal stem cells in the composition according to the present invention is preferably 1 × 10² to 1 × 10⁹ cells/mL, and more preferably 1 × 10³ to 1 × 10⁸ cells/mL. The number of the cells to be administered to a human, though it depends on the number of times of intended administration, usually is in the range of 1 × 10⁵ to 1 × 10⁷ cells/kg body weight per one time of administration. However, the number is not restricted to this range, but may be increased or decreased in accordance with the severity of the symptoms. In particular, even if the number administered is small, those human mesenchymal stem cells will gather at the site of inflammation and thus bring about an excellent effect, since human mesenchymal stem cells exhibit homing to the site of inflammation in arthritis, as was found by the present inventors, whereas those human mesenchymal stem cells which turn out to be no longer needed will disappear spontaneously. Therefore, the doses of human mesenchymal stem cells may be set as desired in a wide range.

### [Examples]

While the present invention will be described in further detail below, it is not intended that the present invention be limited to those examples.

### [Formation of arthritis model mice]

Female BALB/c mice, 4-week old, were purchased from Sankyo Labo Service Corporation (Mishima-shi, Shizuoka) and kept under the SPF (Specific Pathogen Free) condition for 2 weeks. As for the environment, lighting was controlled to on/off at 12-hour intervals, the temperature kept constant, and free access to feed which had been γ-irradiated and water was allowed. As to the manner of keeping animals, the bylaw of the Center of Experimental Animals, Tokyo Medical University was followed. To induce arthritis, a commercially available monoclonal antibody cocktail kit for induction of arthritis (Arthrogen-CIA mAb , Chondrex) was used, which was handled according to the manual attached. Mice which were kept to reach 6-week old and about 20 g of body weight, were peritoneally injected with the anti-type II collagen mixed mouse antibodies (10 mg/mL) which was included in the kit was injected into the tail vein at an amount of 200 µL (2 mg)/mouse (defined as "day 0"). On day 3 after the administration of the antibodies (on day 4 after the administration of antibodies in the case of "Experiment 3" mentioned later), the lipopolysaccharide (LPS) solution (500 µg/mL) which was included in the kit was intraperitoneally administered to the mice at an amount of 100 µL (50 µg/mouse). According to this method, the above anti-type II collagen mixed antibodies administered to a mouse would deposit on the surface of the cartilage, and this would induce activation of complements and infiltration of inflammatory cells, thereby causing onset and progression of arthritis. Further, severe arthritis would be brought about by additional administration of LPS. The collagen-induced arthritis which is developed by this method is utilized as a model of human rheumatoid arthritis.

### [Preparation of rat mesenchymal stem cells]

Male Lew Crj-Tg (CAG/GFP)ys rats (Lew-GFP rats), about 10-week old, were euthanized under over-anesthesia, and a pair of femur and crural bones were taken and washed with Tyrode's solution (mftd. by Sigma). Both ends of the bones were cut off, and the marrow was pushed out using a 18G needle attached to a syringe and suspended in the Tyrode's solution. To this was added about 200 mL of growth medium (Dulbecco/Ham's F12, 1:1 mixed medium (mftd. by HyClone) containing 10 % calf fetal serum, 4 mM alanylglutamine), and after passed through 100 µm nylon mesh (mftd. by Becton & Dickinson), growth medium was further added to make 30 mL in volume. The nucleated cells were counted, seeded onto T flasks (75 m², two flasks) (mftd. by Nunc) at a density of 8 × 10⁴ cells/cm², and cultured at 5 % CO₂, 37° C. Culture was continued for 14 days during which the growth medium was exchanged at intervals of 3-4 days. The growth medium then was removed, and after addition of 4.8 mL/culture container of 0.05 % trypsin-containing 0.53 mM EDTA solution (dissociation agent) (mftd. by Invitrogen), incubation was performed for 5 minutes at 37° C to detach the cells. Following addition of a proper amount of the medium, centrifugation, and removal of the supernatant, the cell pellet thus obtained was suspended again in 20 mL of the growth medium. Approximately 1.94 × 10⁷ rat mesenchymal stem cells were obtained and they were designated as the rat mesenchymal stem cells [P0].

The rat mesenchymal stem cells [P0] were seeded onto fresh culture containers (Cell Factory, mftd. by Nunc) at a density of about 1 × 10⁴ /cm² and cultured under the condition of 5 % CO₂, 37° C. The culture was continued for 9 days, during which the growth medium was exchanged at intervals of 3-4 days. The growth medium then was removed, and after addition of 25 mL of a cell dissociation agent per the culture area of 630 cm², incubation was performed for 5 minutes at 37° C to detach the cells. Following addition of a proper amount of the medium, centrifugation, and removal of the supernatant, the cell pellet thus obtained was suspended again in 40 mL of the growth medium. Approximately 1.64 × 10⁸ rat mesenchymal stem cells were obtained and they were designated as rat mesenchymal stem cells [P1].

The rat mesenchymal stem cells [P1] were seeded onto fresh culture containers at a density of about 1 × 10⁴ cells/cm² and cultured under the condition of 5 % CO₂, 37° C. The culture was continued for 7 days, during which the growth medium was exchanged at intervals of 3-4 days. The growth medium then was removed, and after addition of 25 mL of a cell dissociation agent per the culture area of 630 cm², incubation was performed for 5 minutes at 37° C to detach the cells. The medium was added to make the cell suspension 1050 mL in volume, and the cells were washed on a closed-system automatic cell washer (Cytomate: mftd. by Baxter). After this washing, approximately 1.20 × 10⁹ rat mesenchymal stem cells were obtained in 173 mL of the cell suspension and they were designated as rat mesenchymal stem cells [P2].

The rat mesenchymal stem cells [P2] were seeded onto fresh culture containers at a density of about 1 × 10⁴ cells/cm² and cultured under the condition of 5 % CO₂, 37° C. The culture was continued for 7 days, during which the growth medium was exchanged at intervals of 3-4 days. The growth medium then was removed, and after addition of 25 mL of a cell dissociation agent per the culture area of 630 cm², incubation was performed for 5 minutes at 37° C to detach the cells. The medium was added to make the cell suspension 4200 mL in volume, and the cells were washed on a closed-system automatic cell washer (Cytomate: mftd. by Baxter). After this washing, approximately 4.32 × 10⁹ rat mesenchymal stem cells were obtained in 533 mL of the cell suspension and they were designated as rat mesenchymal stem cells [P3].

The rat mesenchymal stem cells [P3] suspension was centrifuged to separate the cells, and the cells were resuspended in 187 mL of acetated Ringer's solution (PlasmaLyteA : mftd. by Baxter) containing 1.2 % human serum albumin (mftd. by Nihon Pharmaceutical Co., Ltd.). To this was added an equivalent amount of acetated Ringer's solution containing 9 % HSA (human serum albumin), 20 % DMSO (mftd. by Edward), mixed, and the mixture was dispensed, frozen in a program freezer, and stored in the vapor phase liquid nitrogen. These frozen cells were thawed before use and employed as rat mesenchymal stem cells in Experiment 3.

### [Experiment 1] Administration of Human Mesenchymal Stem Cells

Schedule of Administration: Arthritis model mice were administered, within 24 hours after the LPS administration (thus, on day 4 after the antibody administration), with 1 ×10⁶ human mesenchymal stem cells (Poietics™, Cambrex: derived from bone marrow, CD105⁺, CD166⁺, CD29⁺, CD44⁺, CD14⁻, CD34⁻, CD45) suspended in 0.21 mL of acetated Ringer's solution for infusion (PlasmaLyte A) containing 3.7 % DMSO and 0.5 % HSA, which was kept at room temperature, by the route of tail vein of the mice at the flow rate of 1 mL/min. The administration of the cells was carried out to the mice which had been warmed on a warm bath to dilate their vein and were put in mouse holders to constrain their bodies. On day 3 after the first administration of human mesenchymal stem cells (thus, on day 7 after the administration of the antibodies), the mice were administered with human mesenchymal stem cells in the same manner as the first administration (MSC-administration group). The group which was not administered with human mesenchymal stem cells served as the control group. The number of the mice of each group was 10.

### [Experiment 2] Administration of Human Mesenchymal Stem Cells

Schedule of Administration: In order to examine human mesenchymal stem cells for their preventive effect, human mesenchymal stem cells were administered to arthritis model mice; within 24 hours before the administration of the anti-type II collagen mixed antibodies (MSC-administration-before-antibody group), within 24 hours before the administration of the LPS solution (MSC-administration-before-LPS group), and within 24 hours after the administration of the LPS solution (MSC-administration-after-LPS group), respectively. The group which was not administered with human mesenchymal stem cells served as the control group. 0.2 mL of acetated Ringer's solution (PlasmaLyte A) containing 3.7 % DMSO and 0.5 % HSA was administered to the MSC-administration-before-LPS group, MSC-administration-after-LPS group, and the control group, respectively, at the same timing as in the administration of human mesenchymal stem cells made to the MSC-administration-before-antibody group. The number of the mice of each group was 4. Administration of human mesenchymal stem cells to the mice was carried out in the same manner as in Experiment 1.

### [Experiment 3] Administration of Rat Mesenchymal Stem Cells

Schedule of Administration: Arthritis model mice were administered, within 24 hours after the administration of the LPS solution (thus, on day 5 after the administration of the antibodies), with 1 × 10⁶ rat mesenchymal stem cells (Cambrex) suspended in 0.21 mL lactated Ringer's solution for infusion (PlasmaLyte A) containing 3.7 % DMSO and 0.5 % HSA, which was kept at room temperature, by the route of tail vein of the mice at the flow rate of 1 mL/min. The administration of the cells was carried out to the mice which had been warmed on a warm bath to dilate their vein and were put in mouse holders to constrain their bodies. On day 3 after the first administration of rat mesenchymal stem cells (thus, on day 8 after the administration of the antibodies), the mice were administered with human mesenchymal stem cells in the same conditions as in the first administration (MSC-administration group). The group which was not administered with rat mesenchymal stem cells serves as the control group. The number of the mice of the MSC-administration group was 8, and that of the control group was 6.

### [Evaluation of Arthritis]

### <Evaluation Method>

The presence of arthritis was evaluated by visual inspection in a conventional manner (J Exp Med. 2000; 191: 313-320 , Arthritis Rheum. 2007; 56(2): 521-530). The severity of arthritis was quantified by a 5-level scores, 0, 1, 1.5, 2, and 3, based on the degree of swelling of the limbs of the mice. Namely, 0 for a joint which appeared normal, 1 for a joint in which mild swelling was observed, 2 for a joint in which definite swelling was observed, and 3 for a joint in which severe swelling was observed. The score 1.5 was given to a joint whose severity was evaluated to be falling in the middle of the scores 1 and 2 Each of the four limbs was evaluated according to the scores, and the sum of the scores for the four limbs was defined as the severity for the mouse (RA score). The mean of RA scores for mice of each group was referred to as mean RA score.

### <Evaluation of Arthritis in Experiment 1>

In Experiment 1, observation was made for the presence and severity of arthritis, from the day on which the antibodies were administered and up to day 9 after the antibody administration. RA scores for each of the mice observed are shown in Table 1.

### [TABLE 1]

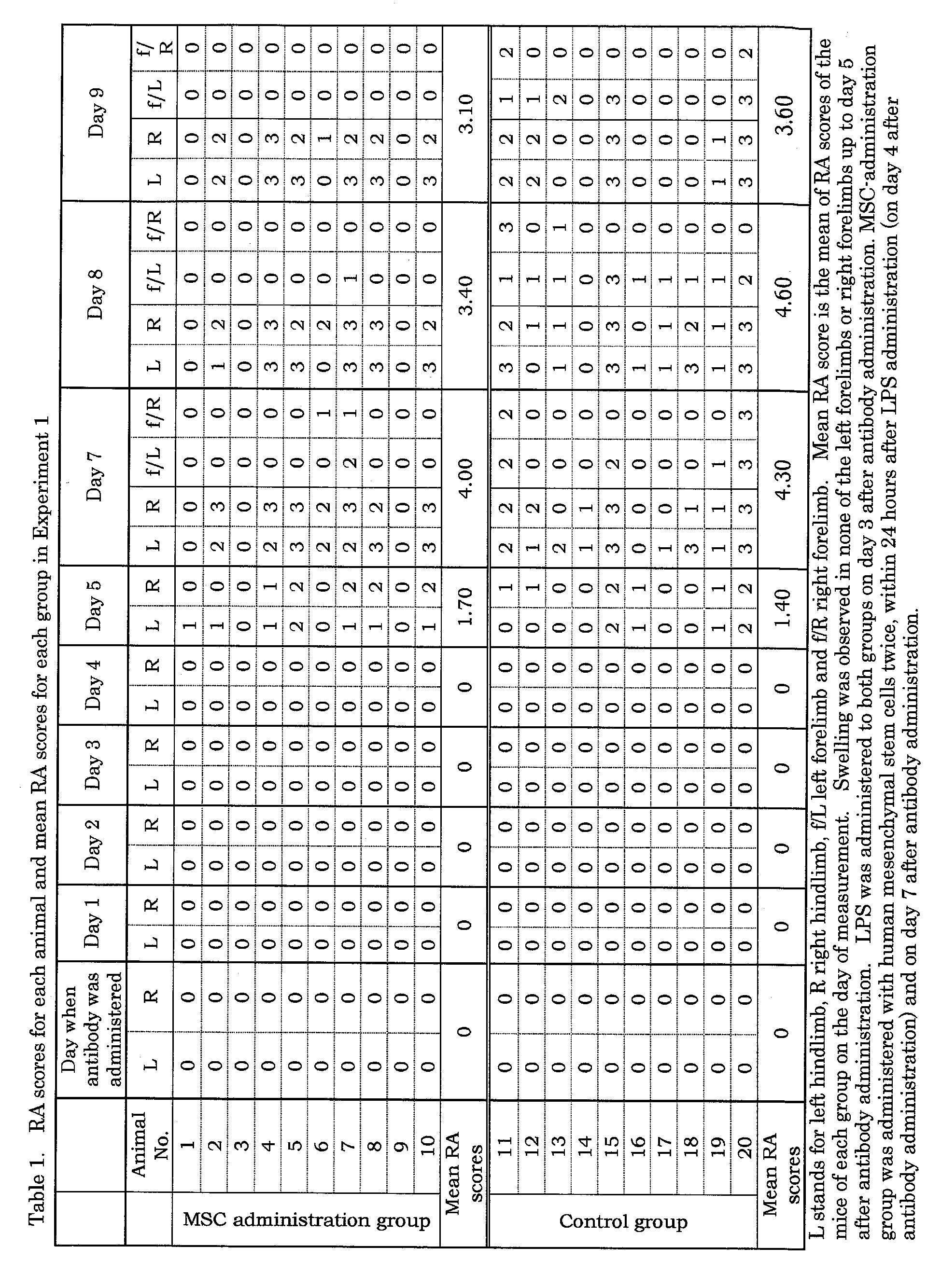

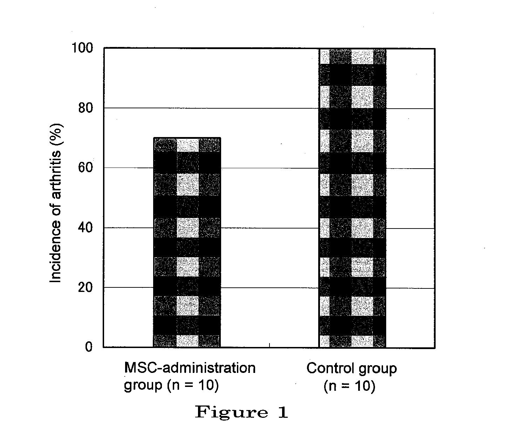

As seen in Table 1, arthritis was observed in none of the animals of the MSC-administration group and the control group within 24 hours after the LPS administration. In the MSC-administration group, arthritis was not observed in 3 out of 10 animals after the second administration of human mesenchymal stem cells (thus, on and after day 7 after the antibody administration) through the observation period, and the incidence of arthritis therefore was 70 % (Fig. 1). On the other hand, all the animals of the control group were observed to have developed arthritis during the above-mentioned period, and thus the incidence of arthritis was 100 % (Fig. 1).

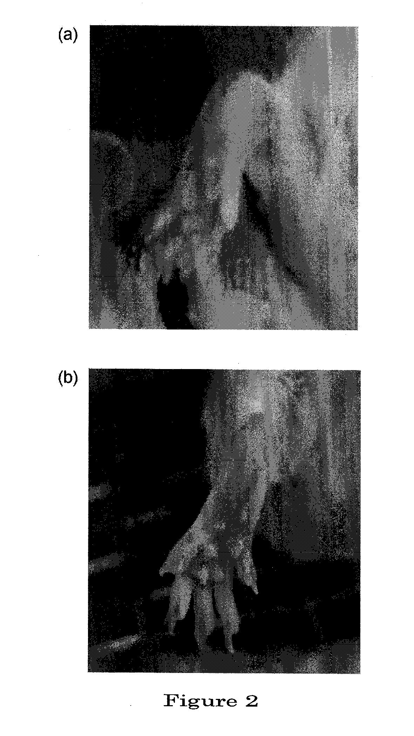

Fig. 2 shows the external appearances of a joint of a mouse of the control group which developed arthritis (Fig. 2a) and of a joint of a mouse of MSC-administration group which develop no arthritis (Fig. 2b). Marked swelling is seen in the control group.

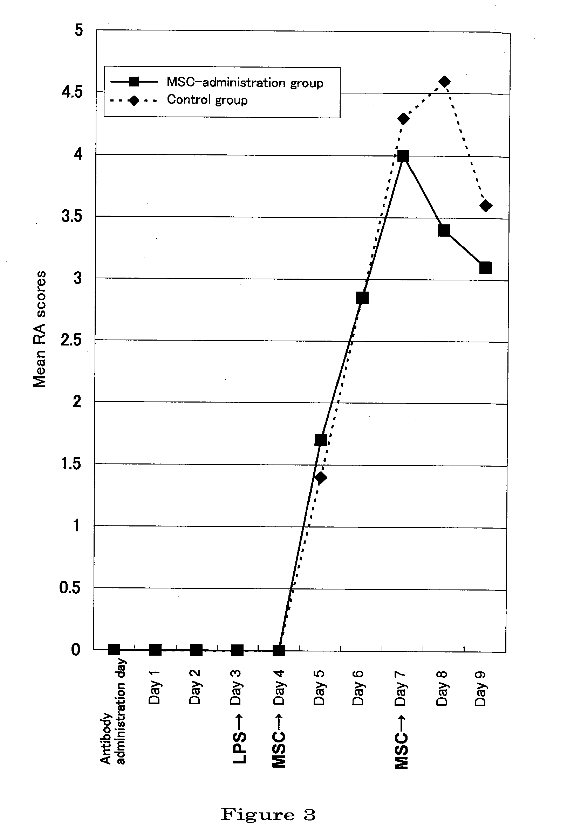

Observation was made for the presence of arthritis and its severity from the day when the antibodies were administered and up to day 9 after the antibody administration, and the results were evaluated based on mean RA scores (Table 1). Comparison of the mean RA scores between MSC-administration group and the control group from the day when the antibodies were administered and up to day 9 showed that the scores on day 8 after the antibody administration were 3.40 for MSC-administration group and 4.60 for the control group, and that those on day 9 after the antibody administration were 3.10 for MSC-administration group and 3.60 for the control group, thus revealing that MSC-administered group showed a remarkably lower mean RA scores on days 8 and 9 after the antibody administration, than those for the control group (Table 1, Fig. 3). Though the mean RA score for MSC-administered group is a little higher on day 5 than that for the control group, it is to be noted that the effect of the administered MSC is not yet reflected on those RA values at this moment of the onset of arthritis, because these are value on the following day after the administration of MSC (thus, day 2 after LPS administration). Further, this model of arthritis is a short-time, acute inflammation model, and the decrease seen in the RA scores for the control group on day 9 after the antibody administration is brought about because the peak of inflammation has gone.

The results presented above demonstrates that human mesenchymal stem cells administered twice suppressed arthritis that was caused to develop by anti-collagen II mixed antibodies and LPS. Furthermore, that fact that no arthritis was observed at all in 3 out of 10 animals of MSC-administration group indicates that MSC exhibited very potent suppressive effect in this intense arthritis model.

### <Evaluation of Arthritis in Experiment 2>

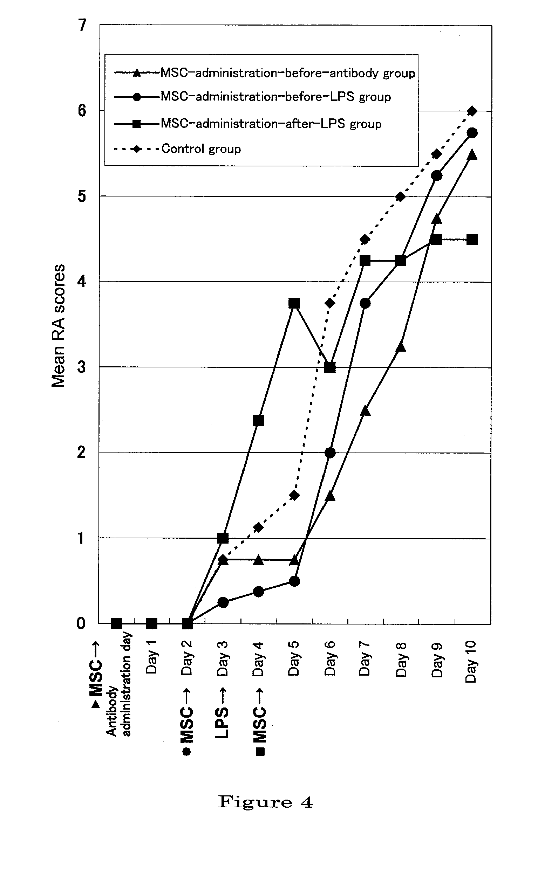

In Experiment 2, observation was made for the presence and severity of arthritis from the day on which the antibodies were administered and up to day 10 after the antibody administration. The results are shown in Table 2 and Fig. 4.

### [TABLE 2]

**Table 2. RA scores for each animal and mean RA scores for each group in Experiment 2**

| | Day when antibody was administered | | Day 1 | | Day 2 | | Day 3 | | | | Day 5 | | | | Day 6 | | | | Day 7 | | | | Day 10 | | | |
|---|---|---|---|---|---|---|---|---|---|---|---|---|---|---|---|---|---|---|---|---|---|---|---|---|---|---|
| | L | R | L | R | L | R | L | R | f/L | f/R | L | R | f/L | f/R | L | R | f/L | f/R | L | R | f/L | f/R | L | R | f/L | f/R |
| MSC-administration-before-antibody group | 0 | 0 | 0 | 0 | 0 | 0 | 0 | 3 | 0 | 0 | 0 | 3 | 0 | 0 | 0 | 3 | 0 | 0 | 0 | 3 | 0 | 0 | 1 | 3 | 1 | 0 |
| | 0 | 0 | 0 | 0 | 0 | 0 | 0 | 0 | 0 | 0 | 0 | 0 | 0 | 0 | 1 | 1 | 0 | 0 | 2 | 1 | 1 | 1 | 2 | 2 | 2 | 1 |
| | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 2 | 1 | 0 | 0 |
| | 0 | 0 | 0 | 0 | 0 | 0 | 0 | 0 | 0 | 0 | 0 | 0 | 0 | 0 | 0 | 1 | 0 | 0 | 0 | 2 | 0 | 0 | 3 | 3 | 1 | 0 |
| Mean RA scores | 0 | | 0 | | 0 | | 0.75 | | | | 0.75 | | | | 1.50 | | | | 2.50 | | | | 5.50 | | | |
| MSC-administration-before-LPS group | 0 | 0 | 0 | 0 | 0 | 0 | 0 | 1 | 0 | 0 | 0 | 0 | 0 | 0 | 0 | 2 | 1 | 0 | 1 | 2 | 2 | 1 | 3 | 2 | 3 | 1 |
| | 0 | 0 | 0 | 0 | 0 | 0 | 0 | 0 | 0 | 0 | 0 | 0 | 0 | 0 | 0 | 2 | 0 | 0 | 1 | 2 | 0 | 0 | 1 | 3 | 0 | 0 |
| | 0 | 0 | 0 | 0 | 0 | 0 | 0 | 0 | 0 | 0 | 0 | 0 | 0 | 0 | 1 | 0 | 0 | 0 | 2 | 0 | 0 | 0 | 3 | 1 | 0 | 0 |
| | 0 | 0 | 0 | 0 | 0 | 0 | 0 | 0 | 0 | 0 | 1 | 1 | 0 | 0 | 1 | 1 | 0 | 0 | 1 | 2 | 1 | 0 | 1 | 3 | 2 | 0 |
| Mean RA scores | 0 | | 0 | | 0 | | 0.25 | | | | 0.50 | | | | 2.00 | | | | 3.75 | | | | 5.75 | | | |
| MSC-administration-after-LPS group | 0 | 0 | 0 | 0 | 0 | 0 | 0 | 1 | 0 | 0 | 2 | 1 | 0 | 0 | 1 | 1 | 0 | 0 | 2 | 1 | 0 | 0 | 1 | 3 | 0 | 0 |
| | 0 | 0 | 0 | 0 | 0 | 0 | 0 | 1 | 0 | 0 | 2 | 1 | 0 | 1 | 2 | 2 | 0 | 1 | 2 | 2 | 0 | 1 | 2 | 2 | 0 | 0 |
| | 0 | 0 | 0 | 0 | 0 | 0 | 0 | 1 | 0 | 0 | 1 | 1 | 1 | 1 | 1 | 1 | 0 | 1 | 1 | 1 | 2 | 1 | 1 | 1 | 2 | 2 |
| | 0 | 0 | 0 | 0 | 0 | 0 | 0 | 1 | 0 | 0 | 1 | 2 | 1 | 0 | 1 | 1 | 0 | 0 | 1 | 1 | 0 | 2 | 1 | 1 | 1 | 1 |
| Mean RA scores | 0 | | 0 | | 0 | | 1.00 | | | | 3.75 | | | | 3.00 | | | | 4.25 | | | | 4.50 | | | |
| Control group | 0 | 0 | 0 | 0 | 0 | 0 | 0 | 0 | 0 | 0 | 0 | 1 | 0 | 0 | 1 | 2 | 0 | 0 | 1 | 2 | 0 | 1 | 1 | 2 | 0 | 1 |
| | 0 | 0 | 0 | 0 | 0 | 0 | 0 | 1 | 1 | 0 | 1 | 1 | 0 | 0 | 1 | 2 | 1 | 0 | 1 | 2 | 2 | 0 | 2 | 3 | 3 | 0 |
| | 0 | 0 | 0 | 0 | 0 | 0 | 0 | 1 | 0 | 0 | 0 | 1 | 0 | 0 | 2 | 2 | 0 | 0 | 2 | 2 | 1 | 0 | 2 | 2 | 2 | 0 |
| | 0 | 0 | 0 | 0 | 0 | 0 | 0 | 0 | 0 | 0 | 1 | 1 | 0 | 0 | 2 | 2 | 0 | 0 | 2 | 2 | 0 | 0 | 3 | 1 | 1 | 1 |
| Mean RA scores | 0 | | 0 | | 0 | | 0.75 | | | | 1.50 | | | | 3.75 | | | | 4.50 | | | | 6.00 | | | |

| | | | | | | | | | | | | | | | | | | | | | | | | | | |
|---|---|---|---|---|---|---|---|---|---|---|---|---|---|---|---|---|---|---|---|---|---|---|---|---|---|---|
| L stands for left hindlimb, R right hindlimb, f/L left forelimb and f/R right forelimb. Mean RA score is the mean of RA scores of the mice of each group on the day of measurement. Swelling was observed in none of the left forelimbs or right forelimbs up to day 2 after antibody administration. LPS was administered to all the groups on day 3 after antibody administration. MSC-administration-before-antibody group was administered with human mesenchymal stem cells within 24 hours before antibody administration. MSC-administration-before-LPS group was administered with human mesenchymal stem cells within 24 hours before LPS administration. MSC-administration-after-LPS group was administered with human mesenchymal stem cells within 24 hours after LPS administration. | | | | | | | | | | | | | | | | | | | | | | | | | | |

Development of arthritis was observed in all the animals of each group. Though notably higher RA scores are seen for MSC-administering-after-LPS group on days 4 to 5 after the antibody administration (thus, the day when MSC was administered and the day following this administration) compared with the control group, this is due to the intense inflammation which had already existed in MSC-administration-after-LPS group before MSC was administered. In the same group, the RA score became lower than that of the control group on day 6 after the antibody administration (thus, day 2 after the MSC administration), which demonstrates a notable suppressive effect of MSC which was administered after the onset of arthritis. In addition, MSC-administration-after-LSP group shows RA scores which are consistently lower than those of the control group thereafter up to day 10 (thus, day 6 after the MSC administration), indicating that MSC, administered only once, suppresses aggravation of arthritis.

Further, the group to which human mesenchymal stem cells were administered in advance within 24 hours before the antibody administration (MSC-administration-before-antibody group) showed lower RA scores compared with the control group during the period of from day 5 to day 10 after the antibody administration (Table 2, Fig. 4). For example, when the mean RA scores are compared between the both groups on days 5 and 6 after the antibody administration, respectively, the scores are 0.75 and 1.5 for MSC-administration-before-antibody group, whereas they are 1.5 and 3.75 for the control group, which shows that the mean RA scores for MSC-administration-before-antibody group are markedly low on both of the days, i.e., 50 % or less compared with those of the control group. This indicates the benefit of prophylactic administration of human mesenchymal stem cells before sensitization occurs.

Furthermore, the group to which human mesenchymal stem cells were administered within 24 hours before the LPS administration (MSC-administration-before-LPS group) also showed lower mean RA scores than those for the control group during the period of from day 3 to day 10 after the antibody administration (Table 2, Fig. 4). For example, when the mean RA scores are compared between the both group on days 5 and 6 after the antibody administration, respectively, the scores are 0.5 and 2.0 for MSC-administration-before-LPS group, whereas they are 1.5 and 3.75 for the control group, which shows that the mean RA scores for MSC-administration-before-LPS group are markedly lower than those for the control group. This result indicates the benefit of prophylactic administration of human mesenchymal stem cells before inflammatory responses are actually elicited, even if sensitization has already been established.

### <Evaluation of Arthritis in Experiment 3>

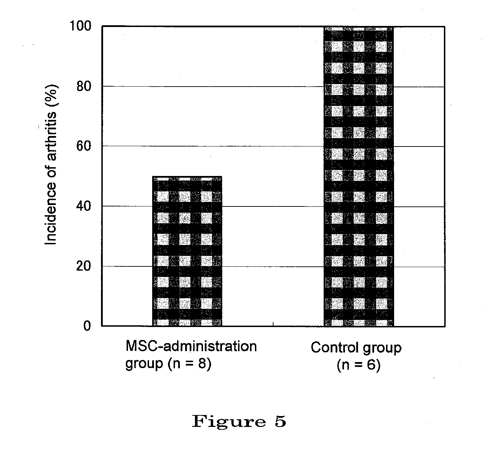

In experiment 3, observation was made for the presence and severity of arthritis from the day on which the antibodies were administered and up to day 11. The results are shown in Table 3 and Fig. 5.

### [TABLE 3]

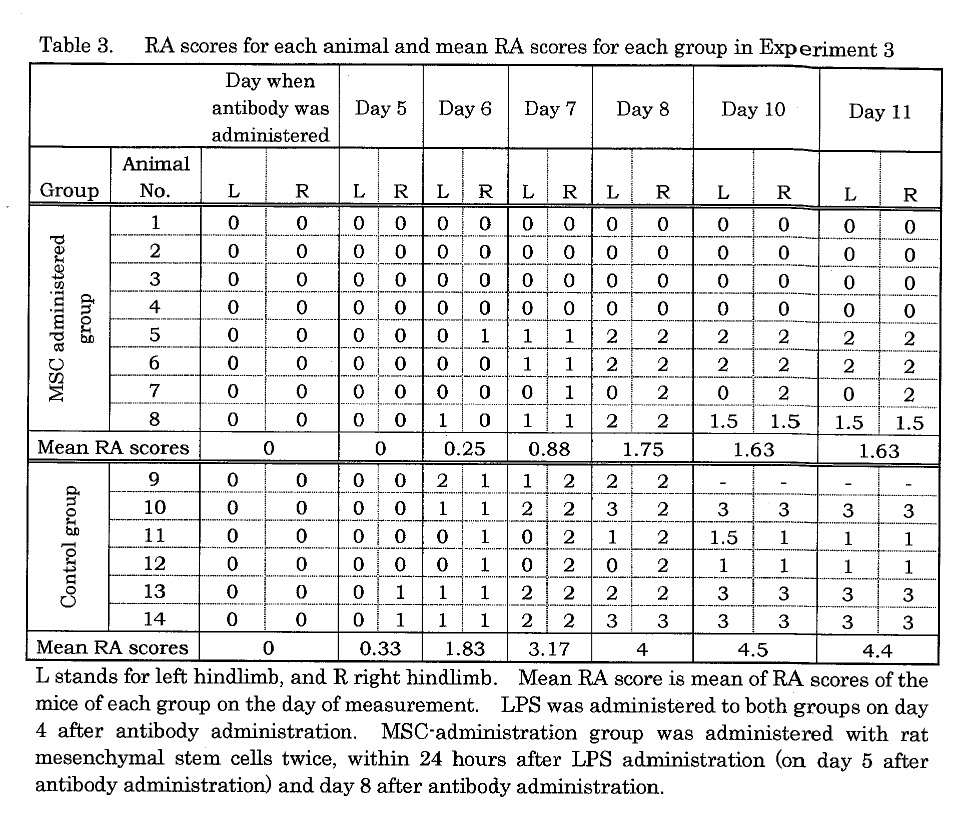

Development of arthritis was observed in all the animals of the control group, and thus the incidence was 100 %. On the other hand, in MSC-administration group, no onset of arthritis was observed in 4 out of 8 animals, and thus the incidence was 50 % (Fig. 5). One animal of the control group died on day 10 after the antibody administration. Relation of the death with arthritis has been unknown.

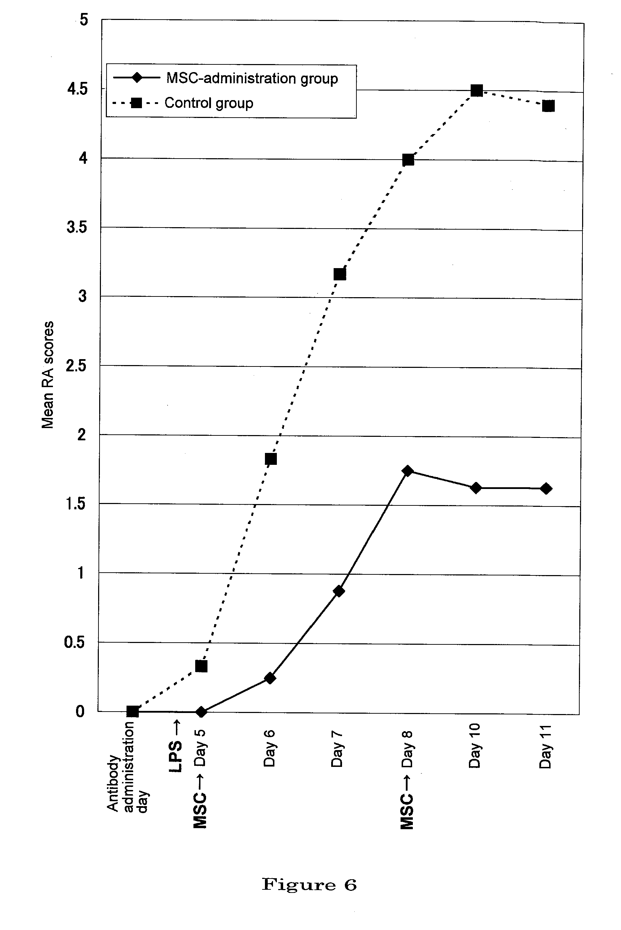

When the mean RA scores are compared between MSC-administration group and the control group during the period of from days 5 to 11 after the antibody administration, MSC-administration group showed a remarkably lower score, 0.25, already on day 6 after the antibody administration (thus, the day which followed the first administration of MSC) than that of 1.83 for the control group (Table 3, Fig. 6). Further, through the period of from day 6 up to day 11 after the administration of the antibody, the mean RA scores for MSC-administration group were remarkably low, i.e., 50 % or less compared with those of the control group. These results indicate that administration of rat mesenchymal stem cells to mice efficiently suppress aggravation of arthritis in the latter. -

### [Preparation of joint and tissue specimen]

The mouse was dissected in the abdomen under anesthesia with diethyl ether (Wako Pure Chemical Industries) and, after the blood was removed from the heart, fixed in 10 % formalin solution (Wako Pure Chemical Industries) for one week. The joint then was excised and placed in a TissueTeck fixation-embedding cassette (TissueTech, Inc.), and after dehydration, embedded in TissueTeck paraffin II60 to form a paraffin block. Five-µm thin tissue sections were prepared, deparaffinized, stained with Mayer's hematoxylin, 1 % eosin, and examined under a microscope.

Preparation of specimen of joint and tissue was carried out on day 14 after the antibody administration in Experiment 1, on day 11 after the antibody administration in Experiment 2, and on day 18 after the antibody administration in Experiment 3.

### [Histological Evaluation in Experiment 1]

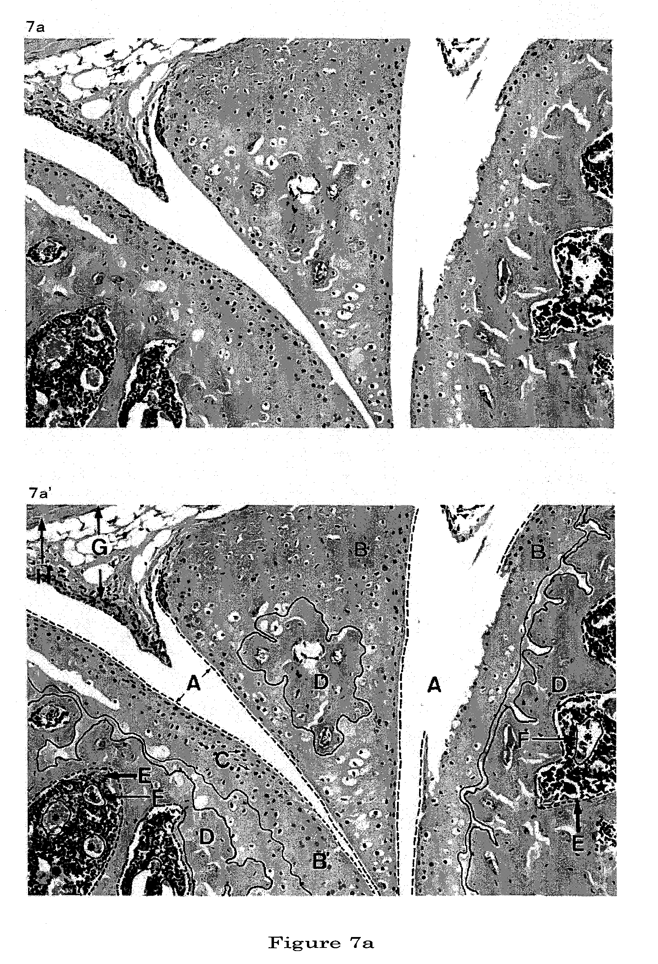

In the tissue section of a normal mice (Fig. 7a), articular cavity (A) was clearly visible. With chondrocytes (C) present in the articular cartilage (hyaline cartilage) (B), ongoing remodeling of the bones was observed. Further, dense-stained, cloud-like bone matrix (cancellous bone) (D) was observed in the deeper layer of the articular cartilage. Bone marrow (E) was present inside of the bone matrix, and in the bone marrow were observed sinusoid (F) consisting of blood vessels as well as hematopoietic stem cells (★). Blood cells (*) were observed in the sinusoid (F).

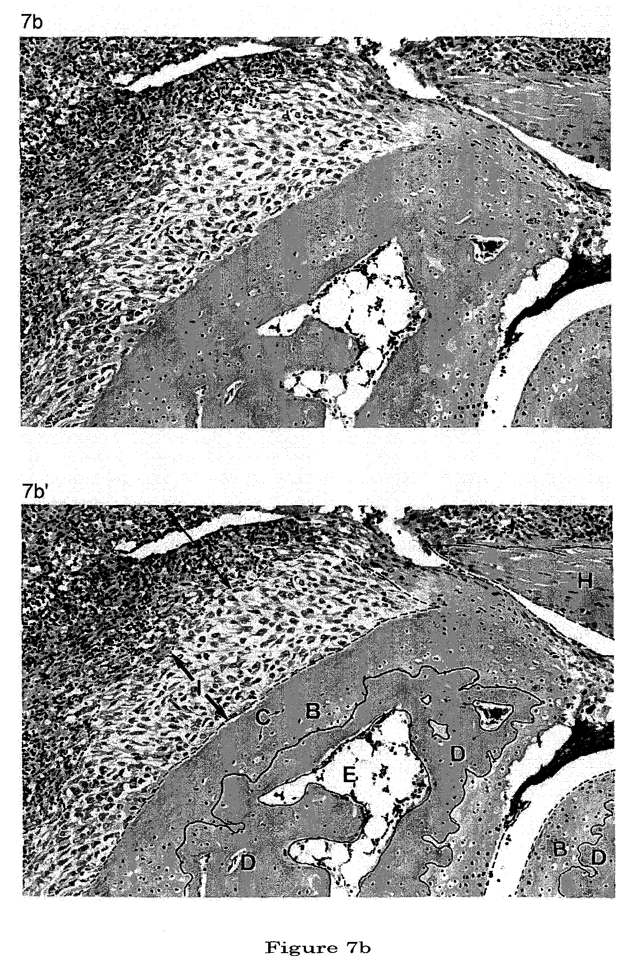

On the other hand, in a tissue section of the site of inflammation in the joint of a mouse of the control group (Fig. 7b), hyperplasia of granulation tissue (I) consisting of synovial cells, fibroblast cells, etc., was observed to have taken place in contact with the articular cartilage (B). And it was found that articular cavity (A) had been lost due to the hyperplasia of the granulation tissue (I). Close to the site of the hyperplasia of granulation tissue, chondrocytes (C) were observed to have decreased in number in the region from superficial to middle layers of the hyaline cartilage (B). Furthermore, a wide range infiltration (J) of inflammatory cells such as polymorphonuclear granulocytes (neutrophils, acidophils), lymphocytes and the like were observed in contact with the site of the hyperplasia of granulation tissues (I). Infiltration of inflammatory cells was observed to have taken place also near the connective tissue (H).

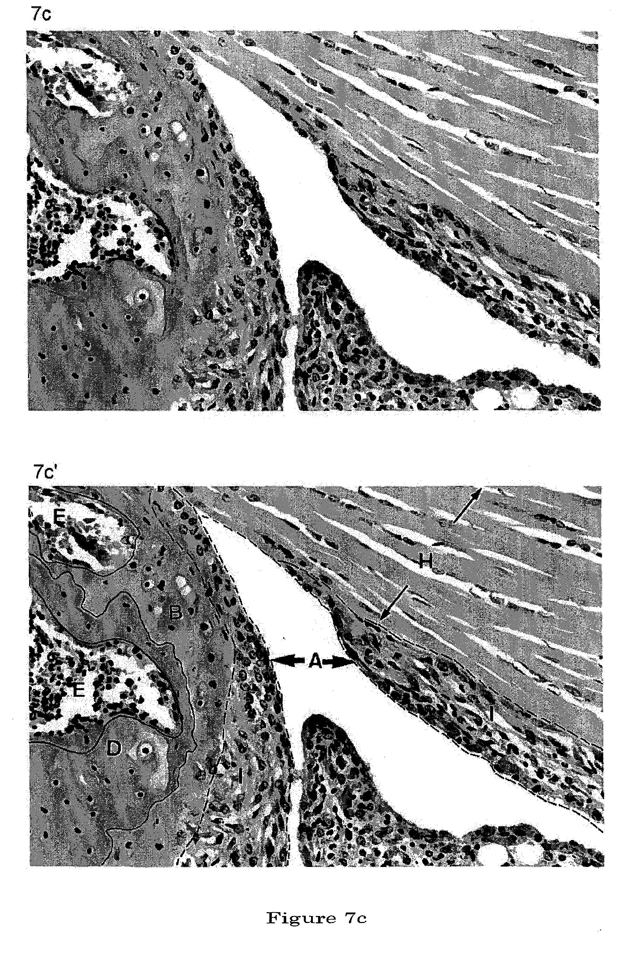

In a tissue section (Fig. 7c) of the site of inflammation in the joint of a mouse of MSC-administration group in which no arthritis was observed according to the RA scores, hyperplasia of granulation tissue (I) was observed to have taken place in contact with the hyaline cartilage (B) and connective tissue (H). The degree of hyperplasia, however, was mild compared with the control group. Neither infiltration of inflammatory cells nor destruction of the bones was observed, and the articular cavity (A) was well maintained. These results demonstrate that the human mesenchymal stem cells which were administered twice suppressed those symptoms which otherwise would have accompany with arthritis, such as destructive abnormalities of the bones, infiltration of inflammatory cells, and hyperplasia of granulation tissue.

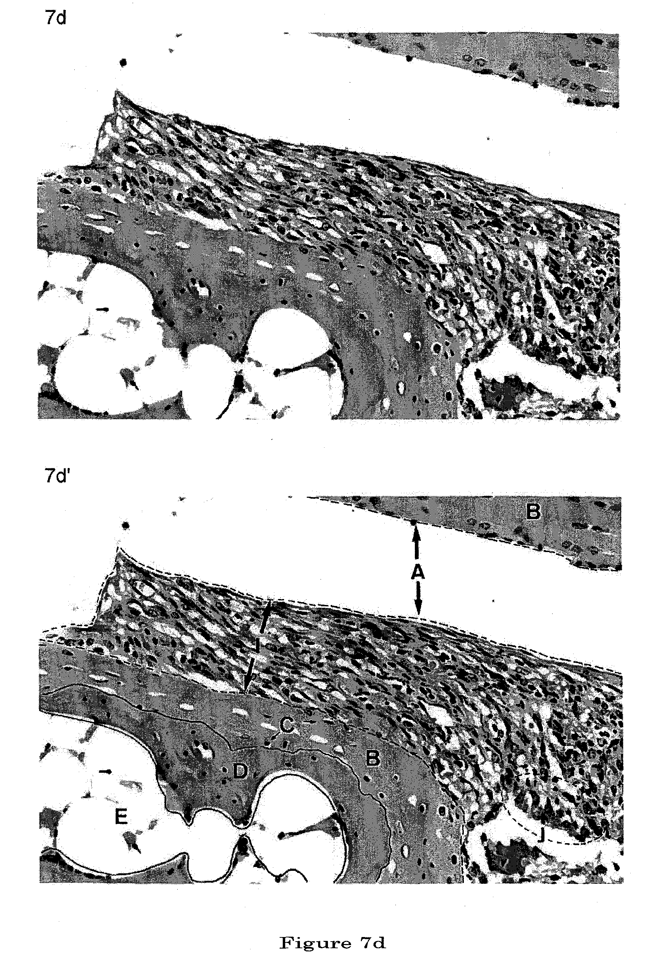

In the tissue section (Fig. 7d) of the site of inflammation of the joint of a mouse of MSC-administration group in which arthritis was observed to have developed according to the RA scores, hyperplasia of granulation tissue (I) was observed in contact with the hyaline cartilage (B) which was located in the lower part of the tissue section. Where hyperplasia of granulation tissue (I) took place, infiltration of inflammatory cells (J) were observed. However, chondrocytes (C) were present in articular cartilage (B) at a level comparable to that in normal mice, and no destructive abnormalities of the bones was observed. Articular cavity (A) was found maintained relatively in good condition. These results demonstrates that human mesenchymal stem cells which were administered twice suppressed symptoms that accompany arthritis, such as destructive abnormalities of the bones, infiltration of inflammatory cells, and hyperplasia of granular tissue in the joint, even in the mice which were observed to have developed arthritis according to their external appearance.

### [Histological Evaluation in Experiment 2]

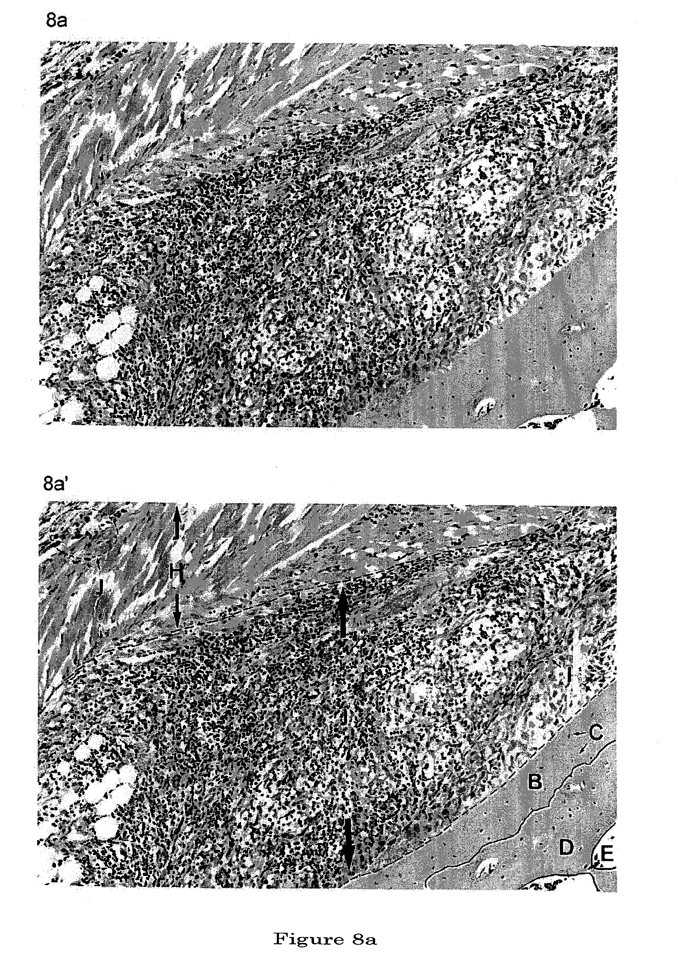

In a tissue section (Fig. 8a) from the control group was observed hyperplasia of granulation tissue (I) in contact with hyaline cartilage (B). Hyperplasia of granulation tissue (I) and a wide range of infiltration of inflammatory cells (J) also were observed between hyaline cartilage (B) and connective tissue (H), having caused the loss of the articular cavity. In the hyaline cartilage (B) close to the site where hyperplasia of the granulation tissue (I) had taken place, chondrocytes (C) had decreased in number in the region from superficial to middle layers, and destructive abnormality of the bones was observed. Infiltration of inflammatory cells (J) was also observed to have taken place near the connective tissue (H).

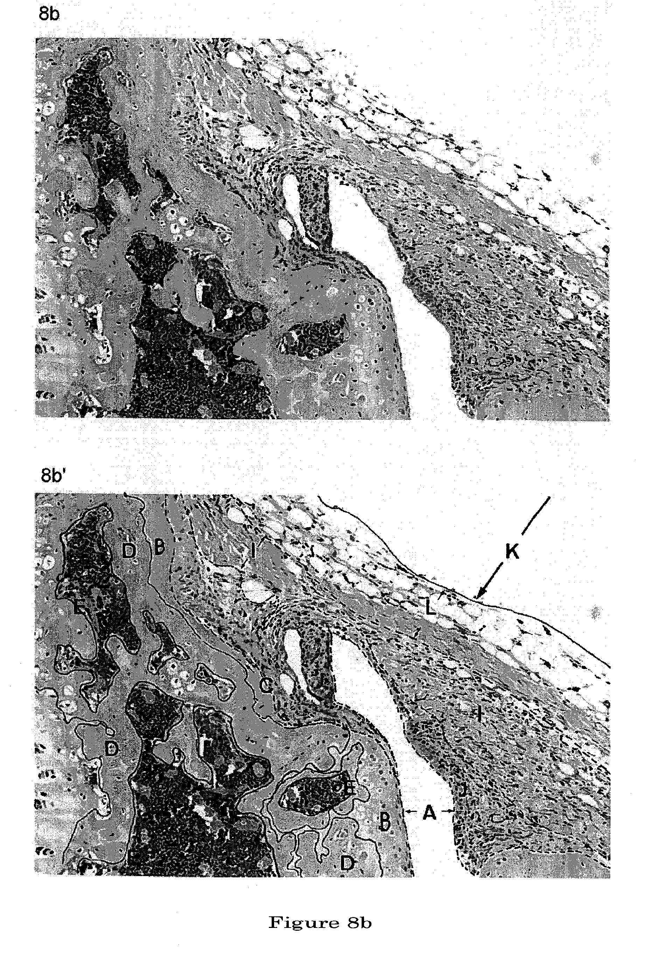

On the other hand, in the tissue section (Fig. 8b) from MSC-administration-before-antibody group, hyperplasia of granulation tissue (I) and infiltration of inflammatory cells (J) were observed between the articular cavity (A) and the adipose tissue (L). However, the articular cavity (A) was maintained well, and no destructive abnormalities of the bones were observed. These results demonstrate that human mesenchymal stem cells, when administered before sensitization by application of the antibodies, therefore before the onset of arthritis, suppresses the symptoms in the joint accompanying arthritis, thus indicating that human mesenchymal stem cells have a prophylactic effect on arthritis.

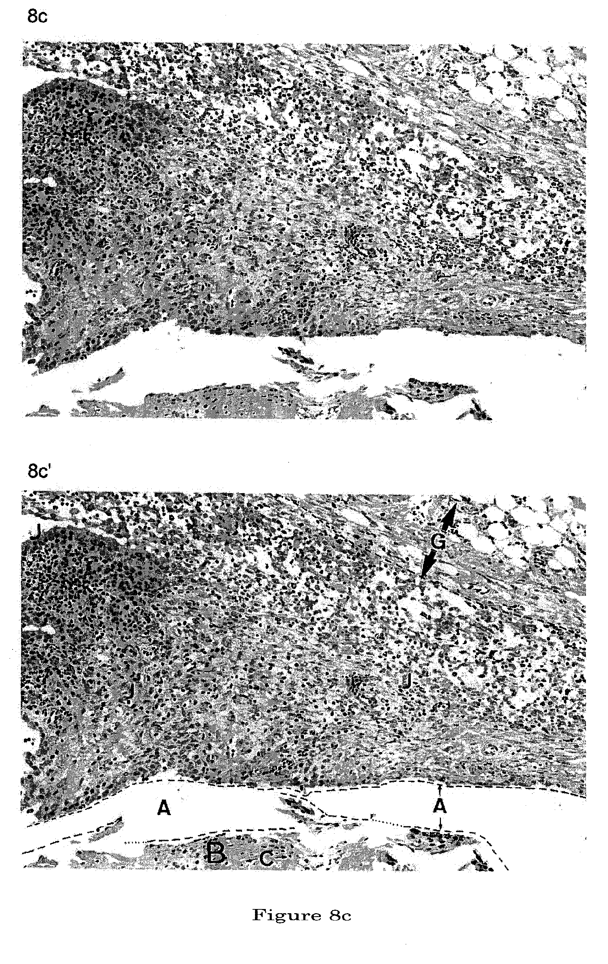

In a tissue section from MSC-administration-after-LPS group (Fig. 8c), infiltration of inflammatory cells (J) were observed between the connective tissue (G) in the upper side of the section and the articular cavity (A). At lower left of the tissue section, chondrocytes (C) were found to have decreased in number in the left end of the hyaline cartilage (B), and a mild destructive abnormalities of the bone was observed. These symptom were milder than those found with the control group. The articular cavity (A) was maintained. These results demonstrate that human mesenchymal stem cells, when administered at an early stage of immunological sensitization before the LPS administration, suppress the symptoms accompanying arthritis, thus indicating that human mesenchymal stem cells are effective on early stage arthritis.

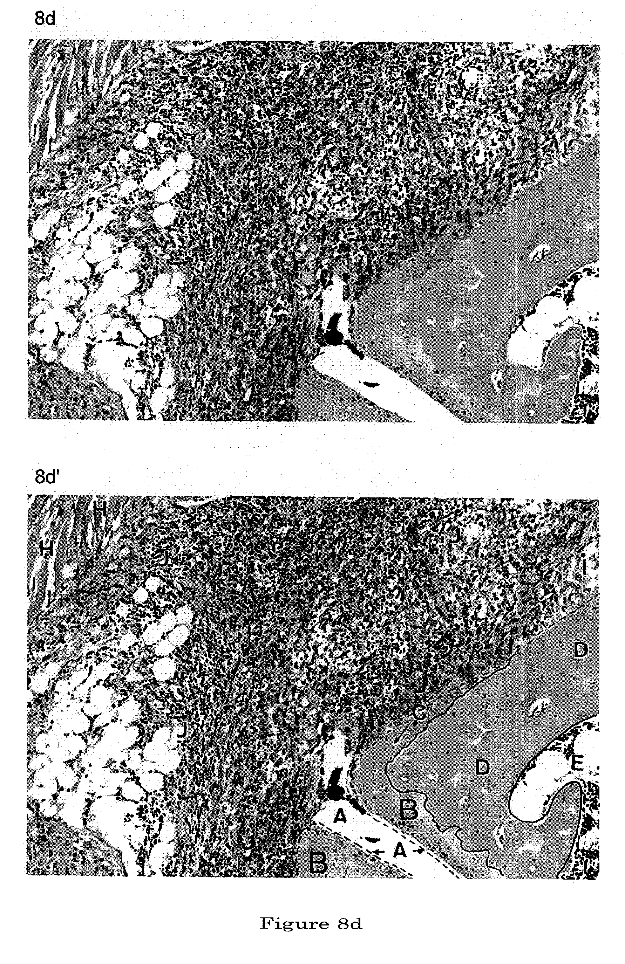

In a tissue section from MSC-administration-after-LPS group (Fig. 8d) was observed hyperplasia of granulation tissue (I) between the connective tissue (H) and hyaline cartilage (B), and a wide range of infiltration of inflammatory cells (J). Chondrocytes (C) were found decreased in number in the hyaline cartilage (B) adjoining the site of hyperplasia of granulation tissue (I) or the site of infiltration of inflammatory cells (J), and destructive abnormalities of the bone were observed. However, these symptoms were milder than those in the control group. Articular cavity (A) was maintained. These results demonstrates that the above symptoms accompanying arthritis are suppressed by a single administration of human mesenchymal stem cells.

### [Histological Evaluation in Experiment 3]

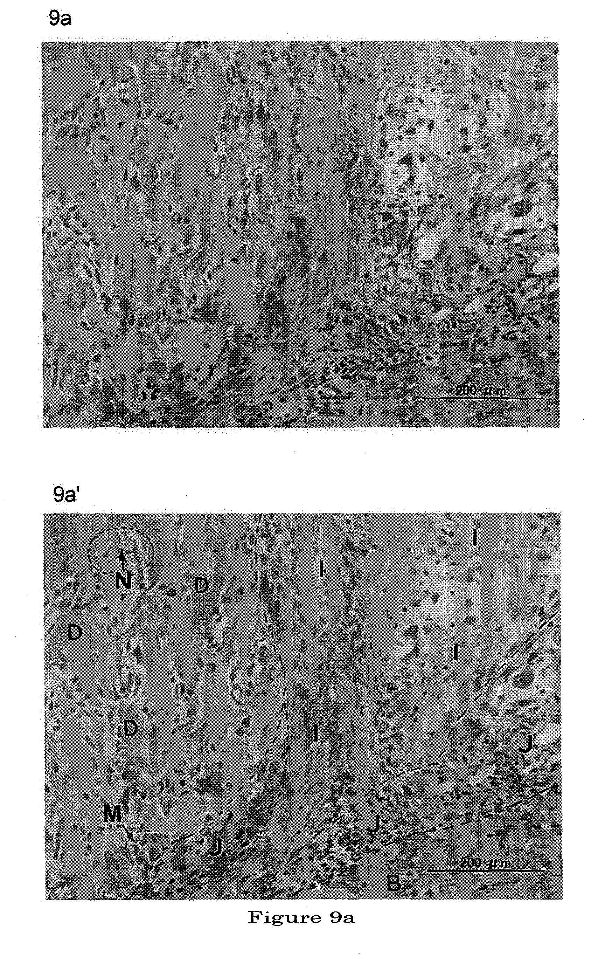

In a tissue section of the site of inflammation in a joint of a mouse of the control group (Fig. 9a), hyperplasia of granulation tissue (I) and infiltration of inflammatory cells (J) were observed in articular cavity (A). Hyaline cartilage (B) was found to have been lost on the bone matrix (D) on the left side of the tissue section, and destructive abnormalities of the bones was observed. Hyperplasia of osteoclasts (M) and increased reactivity of osteoblasts (N) were observed on the bone matrix (D).

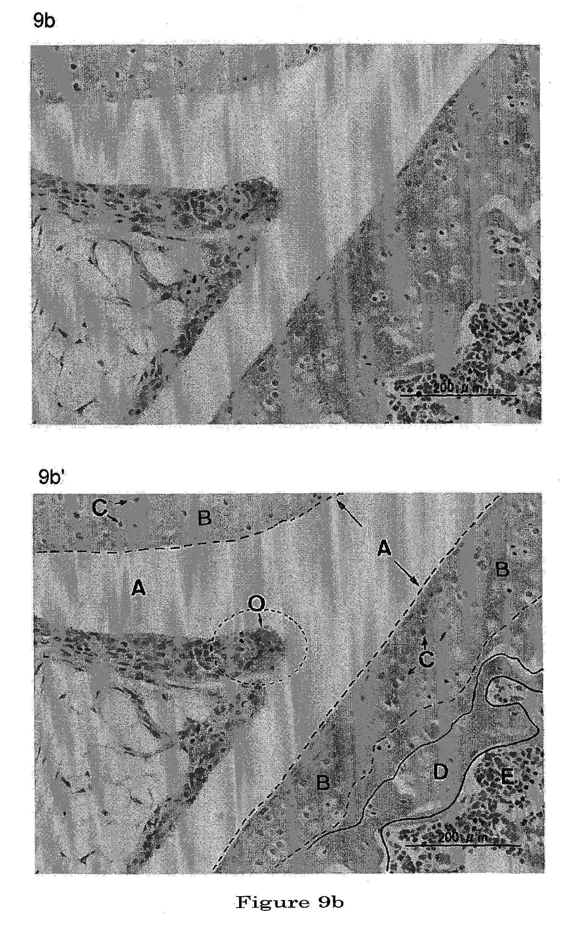

In a tissue section from MSC-administration group (Fig. 9(b)), on the other hand, no abnormality was observed in any of the hyaline cartilage (B), chondrocytes (C), or bone matrix (D), and there was no destructive abnormality of the bones. No infiltration of inflammatory cells was observed, either. Though hyperplasia of synovial epithelium (O) was observed, this was of a mild degree. These results demonstrate that rat mesenchymal stem cells administered thrice suppressed the above symptoms accompanying arthritis in mice.

### [Preparation Example 1] Injection

| | |
|---|---|
| Human mesenchymal stem cells (bone marrow-derived) | 5 × 10⁷ cells |
| Acetated Ringer's solution | to 5 mL |

Human mesenchymal stem cells are suspended in acetated Ringer's solution, and the total volume is adjusted to 5 mL to prepare an injection.

### [Preparation Example 2] Injection

| | |
|---|---|
| Human mesenchymal stem cells (bone marrow-derived) | 1 × 10⁸ cells |
| Sterile physiological saline | to 10 mL |

Human mesenchymal stem cells are suspended in sterile physiological saline, and the total volume is adjusted to 10 mL to prepare an injection.

### [Preparation Example 3] Injection

| | |
|---|---|
| Human mesenchymal stem cells (bone marrow-derived) | 2 × 10⁶ cells |
| 5 % glucose intravenous fluid | to 2 mL |

Human mesenchymal stem cells are suspended in 5 % glucose intravenous fluid, and the total volume is adjusted to 2 mL to prepare an injection.

### INDUSTRIAL APPLICABILITY

The pharmaceutical composition for the treatment and prophylaxis of arthritis comprising human mesenchymal stem cells according to the present invention suppresses and blocks the very inflammation of synovial membrane and suppresses and prevents destruction of the cartilage and bone in mammals, inter alia, in a human. Thus, the present invention can be used as a medicament for the treatment, prophylaxis and prevention of recurrence, of arthritis in mammals, inter alia in a human, and among others rheumatoid arthritis, which affects a great number of patients.

## Claims

1. A pharmaceutical composition for the treatment of arthritis comprising human mesenchymal stem cells.

2. The pharmaceutical composition for the treatment of arthritis according to claim 1, wherein the arthritis is arthritis in a mammal.

3. The pharmaceutical composition for the treatment of arthritis according to claim 2, wherein the mammal is a human or a rodent.

4. The pharmaceutical composition for the treatment of arthritis according to claim 2, wherein the mammal is a human.

5. The pharmaceutical composition for the treatment of arthritis according to claim 3 or 4, wherein the human mesenchymal stem cells are human bone marrow-derived cells.

6. The pharmaceutical composition for the treatment of arthritis according to claim 5, wherein the mammal is a human, and wherein the human is not the same as the human from whom the human mesenchymal stem cells were derived.

7. The pharmaceutical composition for the treatment of arthritis according to claim 5 or 6, wherein the mammal is a human, and wherein the arthritis is rheumatoid arthritis.

8. The pharmaceutical composition for the treatment of arthritis according to one of claims 1 to 7, wherein the composition is an injection.

9. The pharmaceutical composition for the treatment and/or prophylaxis of arthritis according to claim 8, wherein the composition is an injection for intravenous administration.

10. A method for the treatment of arthritis in a mammalian patient, comprising administering to the patient a therapeutically effective amount of human mesenchymal stem cells.

11. The method for the treatment according to claim 10, wherein the mammal is a human or a rodent.

12. The method for the treatment according to claim 10, wherein the mammal is a human.

13. The method for the treatment according to one of claims 10 to 12, wherein the human mesenchymal stem cells are human bone marrow-derived cells.

14. The method for the treatment according to claim 13, wherein the mammal is a human, and wherein the arthritis is rheumatoid arthritis.

15. The method for the treatment according to one of claims 10 to 14, wherein the human mesenchymal stem cells are administered to the patient by injection.

16. The method for the treatment according to claim 15, wherein the human mesenchymal stem cells are intravenously administered.

17. Use of human mesenchymal stem cells for the production of a pharmaceutical composition for the treatment and prophylaxis of arthritis in a mammal.

18. The use according to claim 17, wherein the mammal is a human or a rodent.

19. The use according to claim 17, wherein the mammal is a human.

20. The use according to one of claims 17 to 19, wherein the human mesenchymal stem cells are human bone marrow-derived cells.

21. The use according to claim 20, wherein the mammal is a human, and wherein the arthritis is rheumatoid arthritis.
